(19) Europäisches Patentamt
European Patent Office
Office européen des brevets

(11) **EP 1 918 838 A2**

(12) **EUROPEAN PATENT APPLICATION**

(43) Date of publication:
**07.05.2008 Bulletin 2008/19**

(51) Int Cl.:
***G06F 19/00*** *(2006.01)*

(21) Application number: **07253895.2**

(22) Date of filing: **02.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **05.10.2006 US 544851**

(71) Applicant: **Agilent Technologies, Inc.**
**Santa Clara, California 95051 (US)**

(72) Inventors:
• **Corson, John F.**
**Loveland, CO 80537-0599 (US)**
• **Fulmer-Smentek, Stephanie B.**
**Loveland, CO 80537-0599 (US)**
• **Troup, Charles D.**
**Loveland, CO 80537-0599 (US)**

(74) Representative: **Exell, Jonathan Mark et al**
**Williams Powell**
**Morley House**
**26-30 Holborn Viaduct**
**London EC1A 2BP (GB)**

(54) **Estimation of dynamic range of microarray DNA spike-in data by use of parametric curve-fitting**

(57)    Methods (100), systems (200) and computer readable media for determining the dynamic range of Spike-In controls of microarrays with parameterized sigmoidal curve fitting techniques are disclosed.

Figure 2

EP 1 918 838 A2

**Description**

**BACKGROUND OF THE INVENTION**

Microarray experiments involve multi-step procedures where small differences among samples, techniques, or user-induced variations may alter the microarray data. The microarray community has realized that more quality control is needed in evaluating microarray data. By adding spike-in positive controls, the microarray data can be normalized from one experiment to the next, and hybridization specificity, user-induced variations as well as overall data quality can be addressed for each microarray.

Spike-in controls often comprise a plurality of different transcripts or cDNA in predetermined ratio-generating concentrations that serve as positive microarray controls. Depending on the specific microarray, the spike-ins may be provided as a two-color RNA mixture or a one-color spike-in mixture. The transcripts or nucleotides in a spike-in cocktail have sequences which are designed to hybridize specifically to the control probes of a microarray with minimal self- or cross-hybridization. The spike-in control probes on the microarray are designed to avoid known complementary sequences to biological samples used on most microarrays.

Presently, the most widely used methods for determining the dynamic range and linear region of spike-in microarray data involves basic analysis of signal level to background noise, t-statistic or significance analysis of microarray (SAM) test statistics. There is currently a need in the microarray industry for improved spike-in systems and methods of more efficiently overcoming the difficulties in determining the dynamic range of spike-in controls for microarrays.

**SUMMARY OF THE INVENTION**

Methods for determining the dynamic and linear range of microarray data are provided. Specifically, a method for dynamic range analysis of positive control data for microarrays, comprising providing positive control data (Spike-in data) from a microarray experiment, identifying a plurality of parameters from the positive control data, applying the plurality of parameters to a curve fitting equation, determining a linear range of the positive control data, and applying the linear range of the positive control data to the microarray experiment.

In some embodiments the curve fitting equation of the above method is a sigmoidal curve fitting equation. In particular, embodiments where the sigmoidal curve fitting equation is the following equation.

$$F(x) = \min + \frac{\max - \min}{1 + e^{(-(x-x_0))/w}}.$$

In some embodiments, the plurality of parameters comprise a min, max, x0, and w parameters. The methods may further comprise, identifying the plurality of parameters by optimizing the plurality of parameters with a Levenberg-Marquardt technique. In most methods parameters $x_0$, and w are utilized to determine the linear range of the microarray data.

Some embodiments, also comprise determining the low threshold signal of the spike-in data, and in most instances the low threshold signal error value is determined as well as estimating a signal level for background noise of the spike-in data.

In most embodiments, the methods further comprise estimating a signal for at least one probe which does not bind to a specific target of the microarray experiment. Thus the Spike-in nucleotide sequences are designed so they will not interfere with the hybridization of the sample of interest to the microarray.

The methods of the invention are applicable to both 2-color and 1-color microarray experiments, but in particular 1-color microarrays.

The methods of the invention may comprise converting the Spike-in data in terms of concentration of a given nucleotide (i.e. fmols/micro-liter) to determine the dynamic and linear range of the data.

The invention also provides a computer readable medium carrying one or more sequences of instructions for identifying and selecting a plurality of parameters for analyzing Spike-in data of a microarray, wherein execution of one or more sequences of instructions by one or more processors causes the one or more processors to perform the steps of estimating said plurality of parameters, optimizing the plurality of parameters using a Levenberg-Marquart equation; and using the plurality of optimized parameters in a sigmoidal function to identify the linear range of the Spike-in data. In some embodiments, the computer readable medium further comprises identifying at least one of the plurality of parameters as a minimum signal value for the Spike-in data; and calculating a low threshold error value for the Spike-in data with the minimum signal value.

Other features of the methods of the invention include providing an accurate way to determine the true saturation and noise of the microarray experiment as well as enabling troubleshooting and calibration of a plurality of microarray experiments. The Spike-in data also tests the labeling efficiency and reaction efficiency of a microarray experiment. For example if the Spike-in data does give a saturation point at the top of the Spike-in curve, this suggests that maybe the hybridization reaction time might be too short or the concentration of the Spike-in's needs to be increased. Or if the majority of the Spike-ins do not give a good signal, this may suggest that the dye labeling of the sample may not have been sufficient. The methods of the invention also enable the Spike-ins to give another population of data in which to calculate replicate statistics of a microarray.

An alternative to SAM, the present invention, fills a need in the microarray industry for spike-in systems and methods which require only a single array with a spike-in control set.

The present invention provides methods and systems which overcome the difficulties in determining the dynamic range of spike-in controls of microarrays mentioned above as well as providing advantages in determining the linear range of spike-ins over current methods.

These and other features of the invention will become apparent to those persons skilled in the art upon reading the details of the methods, systems and computer readable media for the estimation of the dynamic range of spike-in microarray data as more fully described below.

## BRIEF DESCRIPTION OF THE DRAWINGS

Embodiments of the present invention will now be described in detail, with reference to the accompanying drawings in which:

Figs. 1A and 1B are illustrations of Intensity vs. Concentration plots of spike-ins of a microarray;

Fig. 2 is a flow chart illustrating one embodiment of the method of the invention;

Fig. 3 is a block diagram illustrating an example of a computer system which may be used in implementing the present invention;

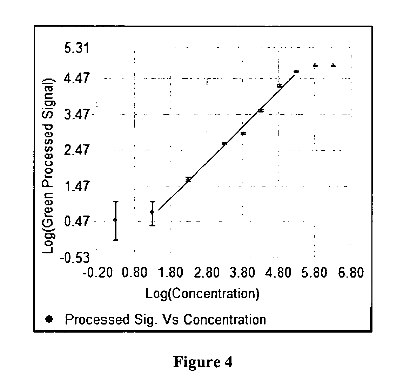

Fig. 4 is a graphical illustration comparing actual data of spike-ins to the fitted data of spike-ins obtained by the methods in accordance with the present invention.

## DETAILED DESCRIPTION OF THE INVENTION

Before the present methods for determining the dynamic range of spike-ins for microarrays are described, it is to be understood that this invention is not limited to particular genes or chromosomes described, as such may, of course, vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within the invention. The upper and lower limits of these smaller ranges may independently be included or excluded in the range, and each range where either, neither or both limits are included in the smaller ranges is also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described. All publications mentioned herein are incorporated herein by reference to disclose and describe the methods and/or materials in connection with which the publications are cited.

It should be noted that as used herein and in the appended claims, the singular forms "a", "an", and "the" include plural referents unless the context clearly dictates otherwise. Thus, for example, reference to "a polynucleotide" includes a plurality of such polynucleotides and reference to "the probe" includes reference to one or more probes and equivalents thereof known to those skilled in the art, and so forth.

The publications discussed herein are provided solely for their disclosure prior to the filing date of the present application. Nothing herein is to be construed as an admission that the present invention is not entitled to antedate such publication by virtue of prior invention. Further, the dates of publication provided may be different from the actual publication dates which may need to be independently confirmed.

## Definitions

The term "nucleic acid" and "polynucleotide" are used interchangeably herein to describe a polymer of any length, e.g., greater than about 10 bases, greater than about 100 bases, greater than about 500 bases, greater than 1000 bases, usually up to about 10,000 or more bases composed of nucleotides, e.g., deoxyribonucleotides or ribonucleotides, or compounds produced synthetically (e.g., PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "ribonucleic acid" and "RNA" as used herein mean a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length. Oligonucleotides are usually synthetic and, in many embodiments, are under 60 nucleotides in length.

The term "oligomer" is used herein to indicate a chemical entity that contains a plurality of monomers. As used herein, the terms "oligomer" and "polymer" are used interchangeably, as it is generally, although not necessarily, smaller "polymers" that are prepared using the functionalized substrates of the invention, particularly in conjunction with combinatorial chemistry techniques. Examples of oligomers and polymers include polydeoxyribonucleotides (DNA), polyribonucleotides (RNA), other nucleic acids that are C-glycosides of a purine or pyrimidine base, polypeptides (proteins), polysaccharides (starches, or polysugars), and other chemical entities that contain repeating units of like chemical structure.

The term "sample" as used herein relates to a material or mixture of materials, typically, although not necessarily, in fluid form, containing one or more components of interest.

The terms "nucleoside" and "nucleotide" are intended to include those moieties that contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The phrase "surface-bound polynucleotide" refers to a polynucleotide "probe" that is immobilized on a surface of a solid substrate, where the substrate can have a variety of configurations, e.g., a sheet, bead, or other structure. In certain embodiments, the collections of oligonucleotide probe elements employed herein are present on a surface of the same planar support, e.g., in the form of an array.

A "labeled population of nucleic acids" refers to mixture of nucleic acids that are detectably labeled, e.g., fluorescently labeled, such that the presence of the nucleic acids can be detected by assessing the presence of the label. A labeled population of nucleic acids is "made from" a chromosome sample, and the chromosome sample is usually employed as template for making the population of nucleic acids.

The term "array" encompasses the term "microarray" and refers to an ordered array presented for binding to nucleic acids and the like.

An "array," includes any two-dimensional or substantially two-dimensional (as well as a three-dimensional) arrangement of spatially addressable regions bearing nucleic acid probes, particularly oligonucleotides or synthetic mimetics thereof, and the like. Where the arrays are arrays of nucleic acids, the nucleic acid probes may be adsorbed, physisorbed, chemisorbed, or covalently attached to the arrays at any point or points along the nucleic acid chain.

Any given substrate may carry one, two, four or more arrays disposed on a front surface of the substrate. Depending upon the use, any or all of the arrays may be the same or different from one another and each may contain multiple spots or features. A typical array may contain one or more, including more than two, more than ten, more than one hundred, more than one thousand, more ten thousand features, or even more than one hundred thousand features, in an area of less than 20 cm$^2$ or even less than 10 cm$^2$, e.g., less than about 5cm$^2$, including less than about 1 cm$^2$, less than about 1 mm$^2$, e.g., 100 $\mu$m$^2$, or even smaller. For example, features may have widths (that is, diameter, for a round spot) in the range from a 10 $\mu$m to 1.0 cm. In other embodiments each feature may have a width in the range of 1.0 $\mu$m to 1.0 mm, usually 5.0 $\mu$m to 500 $\mu$m, and more usually 10 $\mu$m to 200 $\mu$m. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, 20%, 50%, 95%, 99% or 100% of the total number of features). Inter-feature areas will typically (but not essentially) be present which do not carry any nucleic acids (or other biopolymer or chemical moiety of a type of which the features are composed). Such inter-feature areas typically will be present where the arrays are formed by processes involving drop deposition of reagents but may not be present when, for example, photolithographic array fabrication processes are used. It will be appreciated though, that the inter-feature areas, when present, could be of various sizes and configurations.

Each array may cover an area of less than 200 cm$^2$, or even less than 50 cm$^2$, 5 cm$^2$, 1 cm$^2$, 0.5 cm$^2$, or 0.1

cm$^2$. In certain embodiments, the substrate carrying the one or more arrays will be shaped generally as a rectangular solid (although other shapes are possible), having a length of more than 4 mm and less than 150 mm, usually more than 4 mm and less than 80 mm, more usually less than 20 mm; a width of more than 4 mm and less than 150 mm, usually less than 80 mm and more usually less than 20 mm; and a thickness of more than 0.01 mm and less than 5.0 mm, usually more than 0.1 mm and less than 2 mm and more usually more than 0.2 and less than 1.5 mm, such as more than about 0.8 mm and less than about 1.2 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, the substrate may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulse-jets of either nucleic acid precursor units (such as monomers) in the case of *in situ* fabrication, or the previously obtained nucleic acid. Such methods are described in detail in, for example, the previously cited references including US6242266, US6232072, US6180351, US6171797, US6323043, U.S. Patent Application Serial No. 09/302,898 filed April 30, 1999 by Caren et al., and the references cited therein. These references are incorporated herein by reference. Other drop deposition methods can be used for fabrication, as previously described herein. Also, instead of drop deposition methods, photolithographic array fabrication methods may be used. Inter-feature areas need not be present particularly when the arrays are made by photolithographic methods as described in those patents.

An array is "addressable" when it has multiple regions of different probes (e.g., different oligonucleotide sequences) such that a region (i.e., a "feature" or "spot" of the array) at a particular predetermined location (i.e., an "address") on the array will detect a particular target sequence. Array features are typically, but need not be, separated by intervening spaces. It should be noted that the terms "target" and "probe" are sometimes used differently in certain publications.

A "scan region" refers to a contiguous (preferably, rectangular) area in which the array spots, probes or features of interest are found or detected. Where fluorescent labels are employed, the scan region is that portion of the total area illuminated from which the resulting fluorescence is detected and recorded. Where other detection protocols are employed, the scan region is that portion of the total area queried from which resulting signal is detected and recorded. For the purposes of this invention and with respect to fluorescent detection embodiments, the scan region includes the entire area of the slide scanned in each pass of the lens, between the first feature of interest, and the last feature of interest, even if there exist intervening areas that lack features of interest.

An "array layout" refers to one or more characteristics of the features, such as feature positioning on the substrate, one or more feature dimensions, and an indication of a moiety at a given location. "Hybridizing" and "binding", with respect to nucleic acids, are used interchangeably.

. The term "stringent assay conditions" as used herein refers to conditions that are compatible to produce binding pairs of nucleic acids, e.g., probes and targets, of sufficient complementarity to provide for the desired level of specificity in the assay while being incompatible to the formation of binding pairs between binding members of insufficient complementarity to provide for the desired specificity. The term "stringent assay conditions" refers to the combination of hybridization and wash conditions.

A "stringent hybridization" and "stringent hybridization wash conditions" in the context of nucleic acid hybridization (e.g., as in array, Southern or Northern hybridizations) are sequence dependent, and are different under different environmental parameters. Stringent hybridization conditions that can be used to identify nucleic acids within the scope of the invention can include, e.g., hybridization in a buffer comprising 50% formamide, 5xSSC, and 1% SDS at 42°C, or hybridization in a buffer comprising 5×SSC and 1% SDS at 65°C, both with a wash of 0.2xSSC and 0.1% SDS at 65°C. Exemplary stringent hybridization conditions can also include a hybridization in a buffer of 40% formamide, 1 M NaCl, and 1% SDS at 37°C, and a wash in 1×SSC at 45°C. Alternatively, hybridization to filter-bound DNA in 0.5 M NaHPO$_4$, 7% sodium dodecyl sulfate (SDS), 1 mnM EDTA at 65°C, and washing in 0.1×SSC/0.1% SDS at 68°C can be employed. Yet additional stringent hybridization conditions include hybridization at 60°C or higher and 3 × SSC (450 mM sodium chloride/45 mM sodium citrate) or incubation at 42°C in a solution containing 30% formamide, 1M NaCl, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions determine whether a nucleic acid is specifically hybridized to a probe. Wash conditions used to identify nucleic acids may include, e.g.: a salt concentration of about 0.02 molar at pH 7 and a temperature of at least about 50 °C or about 55°C to about 60°C; or, a salt concentration of about 0.15 M NaCl at 72°C for about 15 minutes; or, a salt concentration of about 0.2xSSC at a temperature of at least about 50°C or about 55 °C to about 60°C for about 15 to about 20 minutes; or, the hybridization complex is washed twice with a solution with a salt concentration of about 2×SSC containing 0.1% SDS at room temperature for 15 minutes and then washed twice by 0.1×SSC containing 0.1% SDS at 68°C for 15 minutes; or, equivalent conditions. Stringent conditions for washing can also be, e.g., 0.2×SSC/0.1% SDS at 42°C. In instances wherein the nucleic acid molecules are deoxyoligonucleotides ("oligos"), stringent conditions can include washing in 6xSSC/0.05% sodium pyrophosphate at 37 °C (for 14-base oligos), 48 °C (for 17-base oligos), 55°C (for 20-base oligos), and 60°C (for 23-base oligos). See Sambrook, Ausubel, or Tijssen (cited below) for detailed descriptions of equivalent hybridization and wash conditions and for reagents and buffers, e.g., SSC buffers and equivalent reagents and conditions.

Stringent hybridization conditions may also include a "prehybridization" of aqueous phase nucleic acids with complexity-reducing nucleic acids to suppress repetitive sequences. For example, certain stringent hybridization conditions include, prior to any hybridization to surface-bound polynucleotides, hybridization with Cot-1 DNA, or the like.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

The term "mixture", as used herein, refers to a combination of elements, that are interspersed and not in any particular order. A mixture is heterogeneous and not spatially separable into its different constituents. Examples of mixtures of elements include a number of different elements that are dissolved in the same aqueous solution, or a number of different elements attached to a solid support at random or in no particular order in which the different elements are not specially distinct. In other words, a mixture is not addressable. To be specific, an array of surface-bound polynucleotides, as is commonly known in the art and described below, is not a mixture of capture agents because the species of surface-bound polynucleotides are spatially distinct and the array is addressable.

"Isolated" or "purified" generally refers to isolation of a substance (compound, polynucleotide, protein, polypeptide, polypeptide, chromosome, etc.) such that the substance comprises the majority percent of the sample in which it resides. Typically in a sample a substantially purified component comprises 50%, preferably 80%-85%, more preferably 90-95% of the sample. Techniques for purifying polynucleotides, polypeptides and intact chromosomes of interest are well-known in the art and include, for example, ion-exchange chromatography, affinity chromatography, sorting, and sedimentation according to density.

The terms "assessing" and "evaluating" are used interchangeably to refer to any form of measurement, and include determining if an element is present or not. The terms "determining," "measuring," and "assessing," and "assaying" are used interchangeably and include both quantitative and qualitative determinations. Assessing may be relative or absolute. "Assessing the presence of' includes determining the amount of something present, as well as determining whether it is present or absent.

If a surface-bound polynucleotide "corresponds to" a chromosome, the polynucleotide usually contains a sequence of nucleic acids that is unique to that chromosome. Accordingly, a surface-bound polynucleotide that corresponds to a particular chromosome usually specifically hybridizes to a labeled nucleic acid made from that chromosome, relative to labeled nucleic acids made from other chromosomes. Array features, because they usually contain surface-bound polynucleotides, can also correspond to a chromosome.

The term "MultErrorGreen" or multiplicative error in the green channel of a typical microarray scanner is the noise that is proportional to the signal which has been determined to be about 8%, for example, a 100 count signal has 8 counts of multiplicative noise, and a 10,000 count signal has 800 counts of multiplicative noise.

The term "dynamic range" refers to a portion of microarray data in which the lower limit is at the limit of detection above the noise of the background (e.g.. the signal is statistically significantly different from zero. In general, the signal is a multiple (3x) of the background noise or the p-value of a t-test comparison between signal and background is below a threshold, e.g. 0.01. The upper limit of the dynamic range is the signal level where the signal saturates (e.g. additional concentration of the target does not increase the signal). The dynamic range comprises the range of signals between these extremes.

The term "linear range" refers to a portion of microarray data where the data is linear within the dynamic range of the data. "Linear range" means that a fit of the measured signal intensity versus known spike-in concentrations can be fit with a line when the data is better than a specified measure of linearity (e.g. $R^2$). R-Squared is the statistical measure of how well a regression line approximates the real data. For example, an r-squared of 1.0 (100%) indicates the line fits through the data perfectly. The slope of the data can also measure how linear the data is, since the fitting and the plotting of the data are both completed in log space. Optimally, the data will generate a slope near 1 and a r-squared value near 1.

The term "Spike-in" refers to a positive control nucleic acid which is added to the sample to be analyzed by a microarray experiment. In general, a "Spike-in" is composed of two parts, a probe and a target (positive control) sequence. The target is 'Spiked In' to the sample mixture and placed on the microarray. The probe refers to the strand of DNA attached to the microarray that the target specifically hybridizes to. A "Spike-in kit" comprises a plurality of different nucleic acids, at varying concentrations, allowing a range of signal intensities for these positive controls to be utilized in analyzing the microarray data. For example, a Spike-In kit may comprise 10 unique target (positive control) sequences, and for each of the 10 different Spike-ins there maybe 30 replicated probes on the microarray for a total of 300 probes randomly placed around the microarray that the Spike-ins can hybridize with. The concentration and the quantity of Spike-In nucleic acid added to the sample is known, allowing quantitative analysis of the microarray experiment.

## Methods, Systems and Computer Readable Media

The methods of the invention are described in terms of use with data derived from arrays or microarrays. It should be understood, however, that the invention may be used with any data that carries spike-in control data, including data derived from arrays, polymerase chain reaction (PCR) experiments, cell sorting, or other techniques. The invention is also described in terms of use with DNA-based arrays, and it is contemplated that the invention may be used with data generated from RNA-based arrays as well.

One of the features of many microarray platforms is the ability to "Spike-in" a cocktail of known quantities of specific nucleotides that hybridize against known probes in the microarray, in order to perform a quality check of the array and/or the experiment. The so called "spike ins" give the user data which is useful in identifying possible problems or issues with the hybridization, labeling, array design, sample concentration, etc.

Spike-in samples are designed so that the various nucleotides in the Spike-in sample provide a range of signal intensities to compare to the microarray results. Spike-in probes are replicated across the microarray to evaluate reproducibility of both the signals and the log ratios of microarray data for quality control of microarray experiments. For example, a microarray Spike-in kit, may have 10 different Spike-in sequences or probes, with 30 replicates of each hybridizing probe positioned across a microarray.

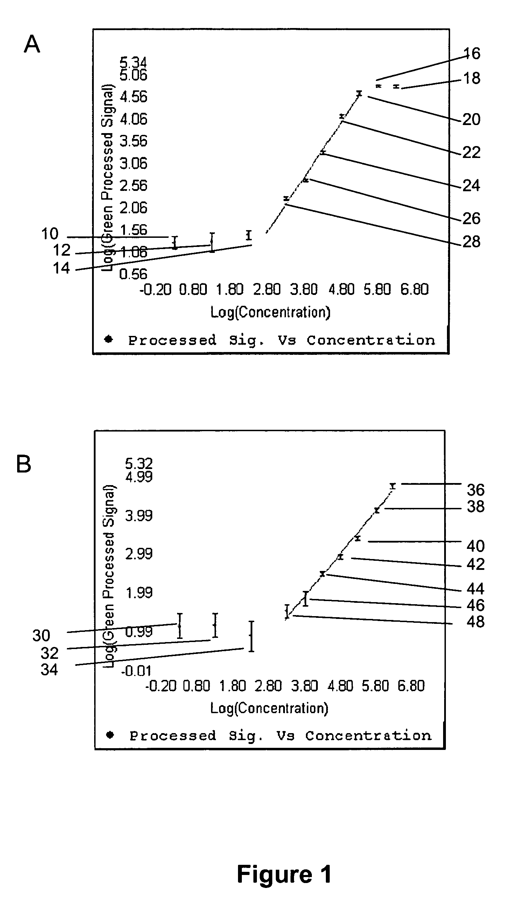

In general, the Spike-in concentration-response of a microarray experiment is represented graphically by a plot of the Log of the Processed Signal (LogProcSig) of the Spike-in nucleic acids vs. the Log of the concentration of each Spike-In utilized in the experiment. The plot is usually sigmoidal, having two asymptotes: one at the scanner saturation point; and one at the level of signal for sequences with no specifically bound target or not enough specifically bound target to generate signal greater than the background noise. Such a plot is shown in Fig. 1A. Other microarrays may produce concentration-response plots which are missing one of the asymptotes, as illustrated in Fig. 1B. The Spike-In sample data shown in Fig. 1A and 1B comprises ten different nucleotides with varying concentrations.

Fig. 1A shows the dose/response curve of the spike-ins from the detection limit 10, 12, 14, to the saturation point 16, 18. Five nucleotides have signal intensity within a linear range 20, 22, 24, 26, 28, while two spike-in nucleotides have intensities 16, 18 which exceed the scanner saturation point, and three of the spike-in nucleotides have signal intensity 10, 12, 14, which is not detectable above noise of the background. At high signal levels the error bars 16, 18, are small since the scanner reaches saturation at this point. Both the signals and standard deviations are underestimated because the saturated data is not excluded from the calculation. At low signal levels the error bars 10, 12, 14, are visible because the signal is dropping into the background noise, also the size of error bars will expand as the signal lowers towards the background noise in a log representation. The signal level at the top of the error bars of the features with lowest signal provides a rough estimate of the lower limit of detection. Signals at this level can be slightly overestimated and the error slightly underestimated because the signals below zero are excluded from the calculation (because they are undefined on a log plot). The most reliable data is found in the signal range where the signal increases linearly 20, 22, 24, 26, 28 with the concentration values of the spike-Ins.

In one embodiment, the method of the invention comprises providing a plurality of unique Spike-ins sequences (for example, 10 different Spike-in controls), and a plurality of probe replicates for each Spike-in (for example, 30 replicate probes specific for each Spike-in control) across a microarray. The method further comprises hybridizing the microarray, scanning and processing to obtain signals from the probes on the microarray and calculating the signal average (Ave) of the Spike-in replicates for each Spike-in sequence as well as the standard deviation(SD) in linear space (representation), followed by converting the data into log space to avoid the undesirable effect of signals below zero being excluded from the data. In this embodiment, the log space representation is still affected when the average value of the replicated Spike-in is below zero, but the signal averages and standard deviation are very accurate when the average signal (Ave) is above zero. The SD in log space may be calculated by:

$$\text{SD in log space} = Log[(AVE + xSD) - (AVE - xSD)]$$

where variable x, in most embodiments, is 1 and the values for AVE (average spike-in probe signals of replicates) and SD (standard deviation of probe signals of replicates) are determined in linear space. If (*AVE - 1SD*) is below zero, the data is censored from the log space plot and (*AVE + 1SD*) is used as the size of the error bar for both the upper and lower error bar of that particular Spike-in signal in log space.

Fig. 1B illustrates a concentration-response plot which has only one asymptote. In this plot, three of the Spike-in nucleotides have low signal levels where the signal is dropping into the background noise 30, 32, 34. Seven Spike-ins appear to be within a linear range 36, 38, 40, 42, 44, 46, 48 while no spike-in nucleotides have signals reaching the scanner saturation point. The curves displayed in the graphs of Figs. 1A and 1B are non-linear, but have a linear behavior within the dynamic range. The line fit shown on the x-axis identifies the linear range of the data. While the Spike-in data shown in Figs. 1A and 1B may appear different in a concentration-response plot, the present invention allows for the dynamic range of the various spike-in data to be determined by modeling the data with certain parameters.

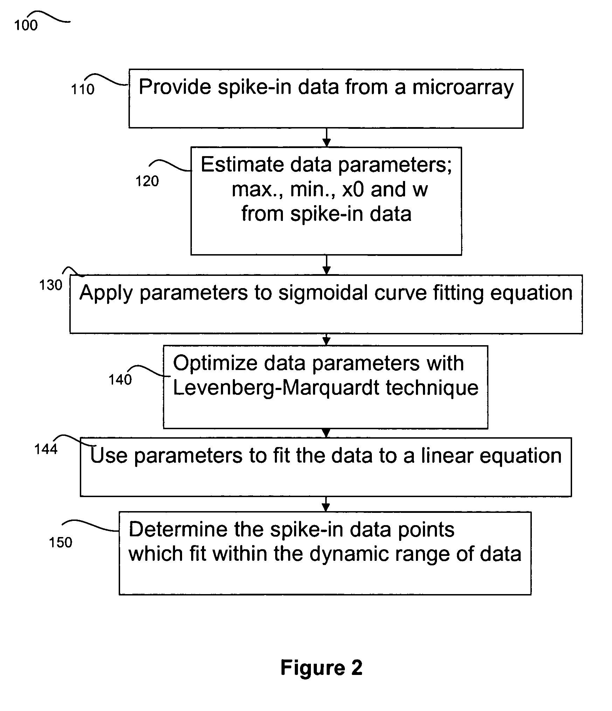

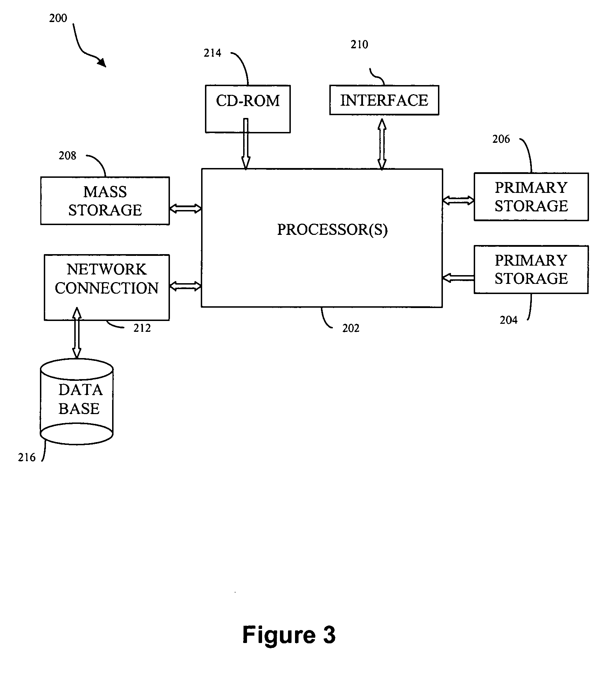

Referring to Fig. 2, there is shown is a flow chart of one embodiment of the subject methods for estimating the dynamic range of microarray DNA Spike-in data utilizing a parametric curve fitting technique. The parametric curve fitting method 100 comprises providing spike-in data from a microarray at event 110. In at least one embodiment, this provision of spike-in data comprises signals obtained from scanning and processing a microarray containing spike-in probes and inputting the spike-in signal data to a system, e.g., such as described with regard to Fig. 3 below, for example.

Event 110 comprises, applying feature extraction software (e.g., Agilent Feature Extraction Software, Agilent Technologies, Inc., Palo Alto, California) which has a list of probes used for Spike-ins. The software comprises multiple spike-in sets and the user can indicate which spike-in set they are using (e.g. 1-color or 2-color or CGH or another), such as by selection via an interactive user interface on a computer display, for example. After extracting the microarray data, the feature extraction software identifies which data comes from the specified probes for the selected spike-ins and this data is utilized in downstream events of the methods of the invention. In most embodiments, the sets of probes used for spike-ins are included in the common controls table in the feature extraction software.

At event 120, estimated values are calculated for selected data parameters from the Spike In data which are useful in estimating the dynamic range. Initially, some statistics are calculated, such as the signal average, Standard Deviation, Percent CV (coefficient of variation) for a plurality of Spike-in replicates. Event 120 may also include estimating the line that fits the linear range of the microarray data. The specific sigmoidal curve fitting parameters identified from the data in event 120, may include but are not limited to, estimates for "Min", "Max", "$x_0$" and "w" parameters. The value "Max" is generally the value for the saturation point of the scanner or the upper limit of detection. Most microarray scanners include this information on the image of the data, i.e. the header of a tiff image of the data. The "Min" value, in general, is the noise background value or the level of signal for sequences with no specifically bound target. The "$x_0$" value is the center of the data (i.e. for $x_0$ the associated signal in signal space is halfway between Min and Max) and is close to the center of the linear range of the data, and "w" is the width of the curve on either side of $x_0$ (in concentration space).

To estimate the parameters in event 120, the following assumptions may be made with regard to the Spike-in data. First, the signal saturation point is assumed to be fixed at or close to the scanner detection limit. For the embodiment shown in Fig. 1, this value is Log(Scanner saturation value) = 4.82. The value of 4.82 derives from the saturation value of the scanner, which in this embodiment is approximately $2^{16}$ represented in log space. The saturation value is dependent on many factors, including but limited to, the type of scanner, or the range of the scan (normal or extended dynamic range). In most embodiments, the asymptotes for the maximum and the minimum signals are not necessarily symmetric, e.g., the upper asymptote may be a function of the scanner offset, while the lower asymptote may be a function of the background noise.

Estimating a starting point for the "Min" value in most embodiments may be completed by calculating the signal average of spike-in replicates for each spike-in sequence on a microarray. For example, the number of Spike-in probes on an array will total 300 when there are 10 different Spike-ins, each with 30 replicate probes spread out randomly across the array. If the value of the average signal of the replicate data points of a Spike-in nucleotide, multiplied by the value of the multiplicative error in the green channel (MultErrorGreen) of a typical scanner is less than the standard deviation of the negative controls (NegCtrls) on the microarray, then a minimum value for the microarray data is obtained and that particular Spike-in is considered part of the minimum value data set.

Negative controls are probes on the microarray which are designed not to bind to any sequence known in the genome or sample being tested, allowing the identification of signal intensities related to nonspecific binding and/or background noise from the equipment. There may be many replicates of these negative controls across the microarray and their population has an average and a standard deviation. The signals associated with the negative controls are considered to be not biological signals. In an optimal noiseless system, the actual value of a probe with nothing bound to it should be zero. Therefore signals close to (within one standard deviation of the negative controls, e.g., within one standard deviation of zero) are considered to be in the noise or not distinguishable from zero. All Spike-in probes have been "spiked in" to generate a real signal, but given multiplicative noise, any Spike-in whose signal average multiplied by the multiplicative noise is less than one standard deviation of the negative controls, is hard to detect given the noise of the system and therefore makes a good candidate probe for use in the determination of the Min value.

Estimating $x_0$ and W at event 120 is completed by starting with the y axis value of (max+min)/2, where "max" is the saturation value, "min" is the noise floor, followed by finding the two closest median log processed signals (MedianLogProcSig), one log processed signal above and one log processed signal below the y-value point, (max+min)/2.

The log concentrations (conc.) for these two points are utilized for determining the slope and intercept for the data. The two data points nearest to (max+min)/2 are utilized to define a line through the spike in data in log space. The calculations for the slope and intercept of the spike-in data allow for the determination of parameters $x_0$ and W.

The slope of the line for a log signal vs. log concentration plot of the spike-in data is generally considered to be the following:

$$Slope = \frac{(HighSignal - LowSignal)}{(High\,RelativeConc - Low\,RelativeConc)}$$

and the intercept is:

$$Intercept = HighSignal - (slope(High\,RelativeConc))$$

where the slope and the intercept are derived from the linear fit, and HighSignal and LowSignal refer to the log processed signals above and below the y-value, (max+min)/2, data point of the Spike-in data, respectively.

After determining the slope and intercept of the line connecting the two Spike-in data points nearest (max+min)/2, the width of the data "w" is determined by applying the following equation:

$$w = 4(slope/(max - min)$$

and once the width of the data is determined, the center of the data "$x_0$" (e.g. the concentration value where the data is half-way between the min and max signals), can be estimated as:

$$x_0 = (max - min)/4w$$

The curve fitting equation of event 130, was chosen based upon looking at the Spike-In data as a sigmoidal function described by:

$$F(x) = min + \frac{max - min}{1 + e^{(-(x-x_0))/w}}$$

where "max" is the saturation value, "min" is the noise floor, "$x_0$" is the center of the data and "w" is the width of data.

In most embodiments, to determine the Low Threshold value of the data, the Min value after the sigmoidal fit, a plurality of Spike-in replicates are averaged for each unique Spike-in sequence to be analyzed on the microarray. The Spike-in replicate averages from the probes discussed above (candidate probes for Min value determination) are used as the starting Min Value for the curve fitting technique, while the final Min Value is gathered after the fitting of the Spike-in data values from the plurality of Spike-in's is complete, and represents the Low Threshold value for the microarray data.

After the estimates of the parameters Min, Max, $x_0$, w are completed in event 120, and subjected to the curve fitting equation in event 130, the data is fit and the parameters (Min, Max, $x_0$, w) are optimized in event 140. In one embodiment the estimated parameters may be adjusted or optimized using the Levenberg-Marquardt technique, allowing the optimization of the parameters from the sigmoidal curve fitting equation to best fit the data. Levenberg- Marquardt algorithm provides a numerical solution to the mathematical problem of minimizing a sum of squares of several, generally nonlinear functions that depend on a common set of parameters. This minimization problem arises especially in least squares curve fitting. The Levenberg-Marquardt algorithm interpolates between the Gauss-Newton algorithm and gradient descent. While the Levenberg-Marquardt technique is preferred in most embodiments, any algorithm which allows optimization of a curve fit using multiple parameters could be utilized.

Once the parameters have been adjusted in event 140, the parameters are used in a linear fitting equation, such as a least square fit, to calculate estimates of the dynamic range and the linear portion of the data in event 144. Estimating the linear range of the data involves using the linear equation and performing a linear fit on the data that has been determined to be 'close to linear'. In the examples shown in Figs. 1 and 2, the model chosen is based upon a sigmoidal function to determine certain parameters and then fitting the data in a linear fashion. While a sigmoidal curve fitting model is preferred in most embodiments, other curve fitting models such as a 4th order polynomial can also be implemented to estimate parameters useful in determining the dynamic range of spike-in data.

Calculating the statistics of the data (High and Low signal; High and Low Concentration, Saturation value, Low Threshold and Low Threshold error) is completed using the data fitting performed above. The Low Relative Concentration is calculated as $x_0 - 2.2(w)$ and the High relative Concentration is calculated as $x_0 + 2.3(w)$. All the points falling between $x_0 - 2.2(w)$ and $x_0 + 2.3(w)$ are fit or modeled through a line with the Slope and R-Squared value reported and are considered to be in the dynamic range of the Spike-in data. The High and Low Signals are calculated from the Sigmoid fit and are considered to be the ends of the linear dynamic range.

The low Threshold error in this embodiment, can be defined by the following:

$$LowThresholdError = \sqrt{\frac{\sum SD(Log(Pr\,ocessedSignals))^2}{A}}$$

The low threshold error is the sum average of the standard deviations squared, of the log processed Spike-in signals of Spike-ins whose AverageSignals* multiplicative Noise are below the standard deviation of the negative controls. For each Spike-in whose replicate average is close to the background noise, i.e. Average(Spike-in(x))(MultErrorGreen) < SD(NegCtrls)), where variable x is the number of replicate probes on a microarray for a particular Spike-in, the standard deviation (SD) of the Log of their processed signals is determined and then the square root of the sum of the squares of the processed signals of these Spike-in's with values close to the background noise are considered to be the Low Threshold Error.

The Low Threshold Error could also be calculated using all data points with values below $(x - 2.3w)$. For each spike-in signal value below this threshold, the standard deviation of the data in linear space can be calculated and then and average of these SD values can be calculated.

The "Max" value may be assumed to be approximately the same as the scanner saturation value when the chemistry of the microarray experiment is known to be carried out under non-saturating conditions. The Min value is determined as described above.

After the parameterized curve fitting routine is completed in event 130 and optimization of the parameters by the Levenberg Marquart technique in 140, the data points in the dynamic range of the Spike-in data are determined or estimated in event 150, by determining the low and high signal, low and high relative concentrations, the slope and the R-squared value derived from the parameterized curve fitting results. These data points can then be used to identify those spike-in probes that are optimized for use on the array and identification of these spike-in probes that provide optimization can be outputted for use by a user. By using $x_0$ as the center of the data, even when the data does not fit a symmetric sigmoidal model, the parameterized curve fitting routine is able to measure the dynamic range of the Spike-in data. With $x_0$ being the center of the data and w is the width of the data, then the upper range of the data is $(x_0 + c_1w)$ and the lower range is $(x_0 - c_2w)$ where $c_1 \neq c_2$, $c_1$ and $c_2$ are multiples of w and are empirically determined to be 2.2 and 2.3, respectively. The values for $c_1$ and $c_2$ are determined to be within a certain percentage of "true linear", e.g. 15-16%. If the spike-in data is analyzed by $c_1w$ or $c_2w$ values away from $x_0$, these data points are considered at the edge of what is identified as linear. In actuality, the Spike-in data is usually never perfectly linear, but the further away from $x_0$ a data point is, in general, the less linear the data is. Thus $c_1$ and $c_2$ are chosen to provide a multiple of w, which is a distance away from $x_0$, where w is non-linear enough to be identified as not in the linear region.

The optimization of parameters for Spike-in microarray data at event 140 and also performed using the parametric curve fitting operation of event 130 will usually be embodied in logic that resides in a computer-readable medium associated with a computer system such as system 200 shown in Fig. 3. The computer system 200 includes any number of processors 202 (also referred to as central processing units, or CPUs) that are coupled to storage devices including primary storage 204 (typically a random access memory, or RAM), primary storage 206 (typically a read only memory, or ROM). As is well known in the art, primary storage 204 acts to transfer data and instructions uni-directionally to the CPU, and primary storage 206 is used typically to transfer data and instructions in a bi-directional manner Both of these primary storage devices may include any suitable computer-readable media containing program elements capable of performing parametric curve fitting operations described above.

A mass storage device 208 is also coupled bi-directionally to CPU 202 and provides additional data storage capacity and may include any of the computer-readable media described above. Mass storage device 208 may be used to store programs, data and the like and is typically a secondary storage medium such as a hard disk that is slower than primary storage. It will be appreciated that the information retained within the mass storage device 208, may, in appropriate cases, be incorporated in standard fashion as part of primary storage 206 as virtual memory. A specific mass storage device such as a CD-ROM 214 may also pass data uni-directionally to the CPU 202.

CPU 202 is also coupled to an interface 210 that includes one or more input/output devices such as such as video monitors, track balls, mice, keyboards, microphones, touch-sensitive displays, transducer card readers, magnetic or paper tape readers, tablets, styluses, voice or handwriting recognizers, or other well-known input devices such as, of course, other computers. Further, data received from a scanner and/or feature extraction system may be inputted to the system 200 via interface 210. CPU 202 optionally may be coupled to a computer or telecommunications network using a network connection as shown generally at 212. With such a network connection, it is contemplated that the CPU 202 might receive information from the network, or might output information to the network in the course of performing the above-described method steps. The above-described devices and materials will be familiar to those of skill in the computer hardware and software arts.

The hardware elements described above may implement the instructions of multiple software modules for estimating the dynamic range of microarray Spike-in data by performing parametric curve fitting operations of this invention. For example, instructions for estimating specific parameters to raw or normalized array data, for determining the dynamic range of Spike-in microarray data using sigmoidal curve fitting functions, and for generating graphical representations of the analyzed Spike-in data, may be stored on mass storage device 208 or 214 and executed on CPU 202 in conjunction with primary memory 206.

In addition, embodiments of the present invention further relate to computer readable media or computer program products that include program instructions and/or data (including data structures) for performing various computer-implemented operations. The media and program instructions may be those specially designed and constructed for the purposes of the present invention, or they may be of the kind well known and available to those having skill in the computer software arts. Examples of computer-readable media include, but are not limited to, magnetic media such as hard disks, floppy disks, and magnetic tape; optical media such as CD-ROM, CDRW, DVD-ROM, or DVD-RW disks; magneto-optical media such as "floptical" disks; and hardware devices that are specially configured to store and perform program instructions, such as read-only memory devices (ROM) and random access memory (RAM). Examples of program instructions include both machine code, such as produced by a compiler, and files containing higher level code that may be executed by the computer using an interpreter.

**EXAMPLE**

The following example is put forth so as to provide those of ordinary skill in the art with a complete disclosure and description of how to make and use the present invention, and is not intended to limit the scope of what the inventors regard as their invention nor is it intended to represent that the experiment below is the only experiment performed. Efforts have been made to ensure accuracy with respect to numbers used (e.g. signal values, averages, etc.) but some experimental errors and deviations should be accounted for.

This example demonstrates one embodiment of the methods for analyzing Spike-in controls for determining the dynamic and linear range of a microarray experiment. Ten Spike-in nucleotides of varying concentrations were added to a microarray sample. There were 30 replicate hybridization probes on the microarray for each of the ten Spike-in controls. Table 1 is a summary of the 300 replicates, 30 replicates for each Spike-in control (probes 1 through 10) tested on the microarray. Table 1 shows the integer ID of each probe tested which is a natural number that is a unique key or identifier in the feature extraction database. The log of the relative concentration, median processed signal, standard deviation of the processed signal, the %CV ((standard deviation/Average signal of a Spike-in) 100), and the signal to noise ratio (signal SNR, which is Average/standard deviation) for Spike-in is also shown in Table 1. The absolute concentrations of the Spike-ins can be difficult to precisely measure, but a Spike-in's relative concentration to the other Spike-ins is easily determined and was used in the method of determining the linear range of the microarray data. The standard deviation of the Negative Controls (SDNegCtls) in this example was 2.71, the Multiplicative Error Green was 0.1 and the Formulation Id for all probes was 2. In the feature extraction database the formulations were tracked. A formulation references the concentration and expected result of each Spike-in that has been determined previously. Depending on the type of application, the same Spike-in's can be reused but with a different formulation. The formulation ID identifies which formulation a given Spike-in's concentration result belongs to. The feature extraction software will know based upon the application (OneColor, TwoColor, or CGH) which formulation to use.

**TABLE 1**

| Integer Id | Probe | LogRelConc | Median Log ProcSignal | Standard Deviation ProcSignal | % CV ProcSignal | Signal SNR |
|---|---|---|---|---|---|---|
| 22 | 1 | 0.30103 | 0.509787 | 0.537449 | 98.8104 | 0.994801 |
| 24 | 2 | 1.30103 | 0.703307 | 0.338339 | 57.9526 | 1.89277 |
| 26 | 3 | 2.30103 | 1.66856 | 0.066142 | 14.5468 | 24.7572 |
| 28 | 4 | 3.30103 | 2.66239 | 0.019894 | 4.6449 | 134.242 |
| 30 | 5 | 3.82607 | 2.94502 | 0.028298 | 6.66392 | 104.1 |
| 32 | 6 | 4.30103 | 3.58682 | 0.028713 | 6.71352 | 125.063 |
| 34 | 7 | 4.82413 | 4.2658 | 0.024955 | 5.86718 | 170.981 |
| 36 | 8 | 5.30103 | 4.66276 | 0.026417 | 6.17865 | 176.571 |
| 38 | 9 | 5.82393 | 4.81442 | 1.25E-05 | 0.002889 | 383702 |
| 40 | 10 | 6.30103 | 4.81442 | 1.39E-05 | 0.003202 | 346260 |

Table 2 shows how the "Average" referred to in obtaining the %CV and signal SNR was calculated. gBgSub-SigAve is a Spike-in's background subtracted signal( the signal produced by the feature extraction software after all of the "background" is removed) and then an average of this adjusted signal was computed for the Spike-in's replicates. Table 2 shows the gBgSubSigAve for eight of the ten Spike-ins in this example to demonstrate how the gBgSubSigAve value was utilized to identify which Spike-ins were utilized for estimating the initial minimum starting value. If the gBg-SubSigAve value of a Spike-in multiplied by the Multiplicative Error Green value, 0.1 was less than the standard deviation of the negative controls, that particular Spike-in was utilized to determine the initial Minimum value estimation, i.e. the Low Threshold value. In this example, probes 1 and 2 were considered to the minimum value estimation but probe 3 was not. 70(.1) < 2.71 so probe 1 was considered for the Minimum point estimation, 5.49(.1) < 2.71 so probe 2 was considered for the min but, 43.87(.1) > 2.71 so probe 3 as well as the rest of the probes were not utilized for the minimum initial estimation.

**TABLE 2**

| Probe Number | gBgSubSigAve |
|---|---|
| 1 | 0.708232 |
| 2 | 5.4946 |
| 3 | 43.8735 |
| 4 | 468.909 |
| 5 | 884.569 |
| 6 | 3907.59 |
| 7 | 18516 |
| 8 | 46268.1 |

Taking the Median Processed Signals of the two considered probes (probes 1 and 2), the Minimum value or Low threshold value was calculated to be:

$$(.510 + .703)/2 = .6065 = \text{Min Value}$$

and the Low Threshold Error was calculated as:

$$SQRT(0.537449^2 + 0.338339^2) = .635$$

The estimation for Max, the saturation value, was log(65502) = 4.81442. The Spike-ins with the lowest and highest Log relative concentration were utilized for the estimation of $X_0$, i.e. $X_0$ = (6.30103 + 0.30103) / 2 = 3.30103, and giving an estimation for w, w = 1. The signal detection limit statistics of the data were determined as the following; saturation point was 4.81, low threshold was 0.44, low threshold error was 0.64 and Spike-in detection limit was 0.94. After inputting the parameters into the Levenberg-Marquart equation, the parameters were optimized to the following values; Min = .44, Max = 4.81, W = .8418, $X_0$ = 3.427.

After determining the above parameters, the parameters were utilized in the curve fitting equation and the results relating to the dynamic and linear range of the Spike-in data are shown in Table 3 below.

**TABLE 3**

| Linear Range Statistics of Log Plot | |
|---|---|
| Low Signal | 0.80 |
| High Signal | 4.59 |
| Low Relative Concentration | 1.49 |
| High Relative Concentration | 5.28 |
| Slope | 1.00 |
| $R^2$ Value | 0.98 |
| **Detection Limit Statistics** | |
| Saturation Point | 4.81 |
| Low Threshold | 0.44 |
| Low Threshold Error | 0.64 |
| Spike-in detection limit | 0.98 |

The Spike-in signal detection limit statistic was calculated as follows; for all of the spike-ins with median log signal less than the low linear signal, calculate in bGSubSignal space the average and standard deviation of each spike-in sequence individually. For each one of these spike-ins, calculate (ave. + 1sd). In summary, determine the median value of the each Spike-in, take the log of that value, and determine the Spike-in detection limit.

While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, scope of the present claims. All such modifications are intended to be within the scope of the claims appended hereto.

"The disclosures in United States patent application no. 11/544851, from which this European patent application claims priority, and in the abstract accompanying this application are incorporated herein by reference."

**Claims**

1. A method for dynamic range analysis of positive control data for microarrays, the method comprising:

    providing positive control data from a microarray experiment (110);
    identifying a plurality of parameters from the positive control data (120);
    applying the plurality of parameters to a curve fitting equation (130);
    determining a linear range of the positive control data (144);
    applying the linear range of the positive control data to select positive control data to be used in the microarray experiment (150); and
    outputting the linear range of the positive control data for use by a user.

2. The method of claim 1, wherein the curve fitting equation is a sigmoidal curve fitting equation (130).

3. The method of any of claim 2, wherein the sigmoidal curve fitting equation is

$$F(x) = \min + \frac{\max - \min}{1 + e^{(-(x-x_0))/w}} .$$

4. The method of any of claims 1-3, wherein the plurality of parameters comprise min, max, $x_0$, and w parameters (120).

5. The method of any of claims 1-4, wherein identifying a plurality of parameters comprises optimizing the plurality of parameters with a Levenberg-Marquardt technique (140).

6. The method of any preceding claim, wherein the positive control data is Spike-In data (110).

7. The method of any claim 6, further comprising determining the low threshold signal of the spike-in data.

8. The method of claim 6 or 7, further comprising determining a low threshold error value of the spike-in data.

9. The method of any of claims 6-8, further comprising estimating a signal for background noise of the spike-in data.

10. The method of any of claims 6 to 9, wherein the Spike-in data tests the labeling efficiency and reaction efficiency of the microarray experiment.

11. The method of any preceding claim, further comprising estimating a signal for at least one probe which does not bind to a specific target of the microarray experiment.

12. The method of any preceding claim, wherein the microarray experiment is a 1-color experiment.

13. A computer readable medium carrying one or more sequences of instructions for identifying and selecting a plurality of parameters for analyzing Spike-in data of a microarray, wherein execution of one or more sequences of instructions by one or more processors causes the one or more processors to perform the steps of:

   estimating said plurality of parameters (120);
   adjusting said plurality of parameters in a sigmoidal function; (130)
   optimizing said plurality of parameters derived from said sigmoidal function using a Levenberg-Marquart equation (140);
   using a linear best fit equation to identify the linear range of the Spike-in data (144); and
   outputting the linear range to a user.

14. The computer readable medium of claim 13, further comprising the steps of;

   identifying at least one of the plurality of parameters as a minimum signal value for the Spike-in data; and
   calculating a low threshold error value for the Spike-in data with the minimum signal value.

15. A system for identifying and selecting a plurality of parameters for analyzing Spike-in data of a microarray, said system comprising:

   a processor (202);
   means for identifying a plurality of parameters from positive control data inputted to said processor (120);
   means for applying the plurality of parameters to a curve fitting equation (130);
   means for determining a linear range of the positive control data (144);
   means for applying the linear range of the positive control data to select positive control data to be used in a microarray experiment (150); and
   a user interface (210) configured to output the linear range of the positive control data for use by a user.

**Figure 1**

100

110 — Provide spike-in data from a microarray

120 — Estimate data parameters; max., min., x0 and w from spike-in data

130 — Apply parameters to sigmoidal curve fitting equation

140 — Optimize data parameters with Levenberg-Marquardt technique

144 — Use parameters to fit the data to a linear equation

150 — Determine the spike-in data points which fit within the dynamic range of data

**Figure 2**

200

214

210

CD-ROM | INTERFACE

206

208

MASS
STORAGE

PROCESSOR(S)

PRIMARY
STORAGE

NETWORK
CONNECTION

PRIMARY
STORAGE

212

202

204

DATA
BASE

216

# Figure 3

**Figure 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5948902 A
- US 6242266 B
- US 6232072 B
- US 6180351 B
- US 6171797 B
- US 6323043 B
- US 30289899 A
- US 11544851 B